Europäisches Patentamt

European Patent Office

Office européen des brevets

(11) Veröffentlichungsnummer: **0 350 692**

**A1**

(12) # EUROPÄISCHE PATENTANMELDUNG

(21) Anmeldenummer: 89111541.2

(22) Anmeldetag: 24.06.89

(51) Int. Cl.⁴: **C12N 15/00 , C12N 9/64**

(30) Priorität: 14.07.88 DE 3823805

(43) Veröffentlichungstag der Anmeldung:
17.01.90 Patentblatt 90/03

(84) Benannte Vertragsstaaten:
**AT BE CH DE ES FR GB IT LI NL SE**

(71) Anmelder: **BASF Aktiengesellschaft
Carl-Bosch-Strasse 38
D-6700 Ludwigshafen(DE)**

(72) Erfinder: **Mueller-Neumann, Markus, Dr.
Wielandstrasse 1
D-6710 Frankenthal(DE)**
Erfinder: **Schmidt, Martin, Dr.
Wichernstrasse 24
D-6803 Edingen-Neckarhausen(DE)**
Erfinder: **Schwarz, Margarete, Dr.
Leinhoehlweg 7
D-6705 Deidesheim(DE)**
Erfinder: **Baldinger, Verena, Dr.
Schiffsgasse 6
D-6900 Heidelberg(DE)**
Erfinder: **Doerper, Thomas, Dr.
Luitpoldstrasse 3
D-6719 Bissersheim(DE)**
Erfinder: **Strube, Karl-Hermann, Dr.
Kurt-Schumacher-Strasse 49 a
D-6720 Speyer(DE)**
Erfinder: **Bialojan, Siegfried, Dr.
Gartenstrasse 34
D-6836 Oftersheim(DE)**

(54) **Polypeptide, ihre Herstellung und Verwendung.**

(57) Es werden Polypeptide der Formel
$tPA_{1-524}$-B
worin $tPA_{1-524}$-B die in der Beschreibung angegebene Bedeutung besitzen, und deren Herstellung beschrieben. Die neuen Polypeptide eignen sich zur Bekämpfung von Krankheiten.

## Neue Polypeptide, ihre Herstellung und Verwendung

Die vorliegende Erfindung betrifft neue Polypeptide mit Plasminogenaktivatoraktivität, Verfahren zu deren Herstellung und deren Verwendung bei der Bekämpfung von Krankheiten.

Der humane, gereifte Gewebe-Plasminogenaktivator (tPA) ist ein Polypeptid aus 527 Aminosäuren und einem Molekulargewicht von etwa 68 kD (Pennica et al. 1983, Nature 301, 214 - 221 und Ny et al. 1984, Proc. Natl. Acad. Sci. USA 81, 5355-5359) (vgl. Abb. 1a-1c). Das Molekül enthält mehrere distinkte Regionen, sog. Domänen, denen verschiedene Funktionen zugeordnet werden. Der N-Terminus wird durch eine Finger-Region gebildet, die dem Fibronektin homolog ist. Anschließend folgt eine Region, die Ähnlichkeit mit mehreren Wachstumsfaktoren besitzt. Im Anschluß daran folgen zwei Kringel-Domänen. Nach einer Spaltstelle, an der das einkettige Molekül in zwei Ketten gespalten werden kann, schließt sich die Protease-Domäne an; diese enthält das aktive Zentrum und besitzt Homologie zu anderen Serin-Proteasen.

Mehrere Eigenschaften machen den Gewebe-Plasminogenaktivator zu einem interessanten Molekül: tPA besitzt eine starke Affinität zu Fibrin, die Plasminogen-Aktivierung ist Fibrin-abhängig; als Protease spaltet tPA Plasminogen zu Plasmin, das wiederum Fibrin zu Spaltprodukten abbaut. tPA ist durch Plasminogenaktivator-Inhibitoren hemmbar. Ferner hat tPA eine relativ kurze Halbwertzeit; das Molekül wird in der Leber rasch abgebaut.

Diese Faktoren bewirken, daß tPA in vivo spezifisch an Blutgerinnseln Plasminogen zu Plasmin aktiviert und durch Abbau des Fibrins die Wiederherstellung des Gefäßstromes bewirkt. tPA kann deshalb in der fibrinolytischen Therapie, z. B. nach einem Herzinfarkt, eingesetzt werden.

Es wurde nun gefunden, daß Polypeptide der Formel

tPA$_{1-524}$-B,

worin

tPA$_{1-524}$ die Aminosäuren 1-524 des humanen, gereiften Gewebe-Plasminogenaktivators und

B eine der Aminosäuresequenzen

- LE
- PW
-            LENPDQADSDGDGRGDACDDIDNDGIPDEDNCPG
- LETRWVSMKKTTMK
- PWGECCGRCLPS
- LEKRLEVDIDIKIRS
- LEGIGAVLKVLTTGLPALISWIKRKRQQG
- LEHHLGGAKQARDV
- PWRGDTPKPQSHNDGDFEEIPEEYLQ

bedeuten, sowie deren Allelvarianten oder Derivate mit am N- und/oder C-Terminus von B um ein bis sechs Aminosäuren verkürzten oder verlängerten Sequenzen verbesserte Eigenschaften besitzen.

Die neuen Polypeptide sind also am C-Terminus des tPA verändert.

Die Erfindung betrifft weiter DNA-Sequenzen, die für die mutierten Polypeptide codieren, sowie Vektoren, die diese DNA-Sequenzen enthalten.

Die neuen Polypeptide lassen sich gentechnisch nach bekannten Verfahren herstellen. Ausgangspunkt für die hier beschriebenen Konstruktionen ist ein cDNA-Klon, im folgenden pUCtpa genannt, der die codierende Sequenz von tPA und noch 5'- und 3'- nicht translatierte Bereiche enthält. Nach einer "leader"- und Prosequenz beginnt das reife Protein mit Ser(1) und endet nach Pro(527).

pUCtPA wird wie folgt isoliert: Aus menschlichem Uterus-Gewebe wird mRNA isoliert und in doppelsträngige cDNA umgeschrieben wird. Nach Einsetzen dieser cDNA in den kommerziell erhältlichen Klonierungsvektor pUC9 wird eine cDNA-Bibliothek angelegt. Die dabei verwendeten Methoden sind beispielsweise in Maniatis et al., Molecular Cloning, CSH-press, nachzulesen. Auch das "screening" solcher Genbanken mit radioaktiv markierten Oligonukleotidsonden ist inzwischen ein vielfach verwendete und beschriebene Methode. Nach diesem Verfahren kann ein cDNA-Klon, der die kodierende Region und angrenzende Bereiche enthält, isoliert werden.

Teile der DNA-Sequenz von pUC9tPA sind mit Hilfe von Restriktionsenzymen leicht zugänglich. Diese Fragmente, ggf. in Verbindung mit chemisch synthetisierten Oligonukleotiden, Adaptoren oder Genfragmenten können benutzt werden, um die DNA-Sequenzen, die für die neuen Polypeptide codieren, zu klonieren. Der Einbau der Genfragmente bzw. synthetischen DNA-Sequenzen in Klonierungsvektoren, z. B. die handelsüblichen Plasmide pBR322, pUC8 oder 9, pUC18 oder 19, M13mp18 oder M13mp19 erfolgt in bekannter Weise. Auch können die Gene oder Genfragmente mit geeigneten chemisch synthetisierten oder aus Bakterien, Phagen, Eukaryontenzellen oder deren Viren isolierten Kontrollregionen versehen werden, die die Expression der Proteine ermöglichen. Die Transformation bzw. Transfektion der so erhaltenen Hybridplasmide in geeignete Wirtsorganismen ist ebenfalls bekannt und eingehend beschrieben. Auch können die Hybridplasmide mit entsprechenden Signalsequenzen versehen werden, die die Sekretion der Polypeptide ins Medium erlauben.

Bei der Expression in Säugerzellen kann man Vektoren verwenden, die das zu exprimierende Gen, in diesem Fall die mutierte tPA-cDNA, unter die Kontrolle des Maus-Metallothionein- oder des viralen SV40-Promoters setzt. Notwendig für die Expression ist das Vorliegen des Methionin-Startcodons und der Leader-/Prosequenz des tPA-Gens. Man isoliert dann Klone, die Kopien dieser Vektoren als Episome oder ins Genom integriert besitzen. Besonders vorteilhaft sind z. B. die Integration und Expression des Fremdgens auf der Basis des Rinderpapillom-Virus. In Verbindung mit prokaryontischen Sequenzen, die für die Replikation in Bakterienzellen und eine Antibiotika-Resistenz codieren, ist der Aufbau sog. "shuttle"-Vektoren möglich. Konstruktion und Vermehrung des Plasmids erfolgen zunächst in Bakterienzellen; anschließend erfolgt die Umsetzung in die Eukaryontenzellen, z.B. in die Maus-Fibroblastenzellinie C127. Es ist selbstverständlich, daß auch andere Zellsysteme, z.B. Hefe, Insektenzellen und andere Säugerzellen, wie z.B. CHO-, L- und 293-Zellen, für die Expression verwendet werden können.

Diese eukaryotischen Expressionssysteme besitzen den Vorteil, daß sie in der Lage sind, ihre Produkte effektiv und meist in nativer Form zu sezernieren. Ferner besitzen sie die Fähigkeit, ihre Produkte posttranslational zu modifizieren.
So erhält der Gewebe-Plasminogenaktivator bei der Expression in Eukaryontenzellen noch Glykosidseitenketten an den Aminosäuren 117, 184 und 448 (Asn). Bakterien sind nicht in der Lage, Glycosidseitenketten zu synthetisieren. Die meisten in Bakterien exprimierten eukaryotischen Proteine, wie auch tPA und die von ihr erfindungsgemäß abgeleiteten Polypeptide, fallen in der Zelle als denaturierte Einschlußkörper an und müssen proteinchemisch renaturiert werden. Ferner sind Bakterien oft nicht in der Lage, die Initiatoraminosäure Methionin vom fertigen Protein abzuspalten. Durch die Verwendung von Sekretionssystemen können diese Schwierigkeiten umgangen werden.

Aufgrund der Degeneration des genetischen Codes ist es aber auch möglich, andere DNA-Sequenzen, z.B. chemisch synthetisierte Gene mit unterschiedlicher DNA-Sequenz für die Expression der neuen Polypeptide zu benutzen.

Die Reinigung der neuen Polypeptide erfolgt durch deren Abtrennung aus dem Kulturmedium durch Affinitäts- und Ionenaustausch-Chromatographie nach bekannten Verfahren.

Die erfindungsgemäß erhaltenen Polypeptide besitzen eine erhöhte Gerinnselspezifität, längere Halbwertzeit, verringerte Inhibitorbindung und/oder größere proteolytische Aktivität und können daher zur Thrombolyse eingesetzt werden. Sie zeigen dabei verbesserte Eigenschaften gegenüber humanem Gewebe-Plasminogenaktivator.

Gegenstand der Erfindung sind daher auch Arzneimittel, die mindestens eines der neuen Polypeptide enthalten, ggf. in einem pharmazeutisch verträglichen Träger oder Bindemittel. Die Arzneimittel können auch Kombinationen der neuen Proteine mit anderen Fibrinolytika, wie Prourokinase, Urokinase oder Streptokinase oder deren Derivate, sowie mit anderen Pharmaproteinen, z. B. Superoxiddismutase enthalten. Weitere Ausgestaltungen der Erfindung sind in den folgenden Beispielen näher beschrieben.
Für gentechnische Methoden sei dazu z. B. auf das Handbuch von Maniatis et al., Molecular Cloning, Cold Spring Harbor Laboratory, 1982, verwiesen.

Beispiel 1

Isolierung eines cDNA-Klones für humanen Gewebe-Plasminogenaktivator

30 g tPA-produzierendes menschliches Uterus-Gewebe wurde in 6 M Guanidiniumthiocyanat, 5 mM Natriumcitrat (pH 7,0), 0,1 M 2-Mercaptoethanol, 0,5 % Sarcosyl im Ultra-Turax® aufgeschlossen. Grobe Zelltrümmer wurden bei 3000 rpm in einem Sorvall-SS34-Rührer (Dupont) abzentrifugiert. Die RNA wurde durch Zentrifugation durch ein 5,7 M CsCl-Kissen über Nacht bei 20 °C und 35.000 rpm in einem SW40-Rotor (Beckman Instruments) abgetrennt. Anschließend wurde die p olyA$^+$-enthaltende RNA-Fraktion durch Affinitätschromatographie an oligo(dT)-Cellulose abgetrennt. Mit Hilfe des Enzyms AMV-Reverse Transcriptase und oligo(dT)12-18 als Starter wurde die polyA$^+$-RNA in einsträngige cDNA umgeschrieben.
Die Synthese des zweiten Stranges erfolgte mit E. coli-DNA-Polymerase I. An die doppelsträngige cDNA wurden mit Hilfe des Enzyms T4-DNA-Ligase Sall-Linker angesetzt. Das kommerziell erhältliche Plasmid pUC9 wurde mit dem Restriktionsenzym Sall linearisiert. Beide DNAs wurden miteinander ligiert und mit dem so erhaltenen Hybrid C aCl$_2$-behandelte, kompetente Zellen des E. coli-Stammes HB101 transformiert.
Die Zellen wurden auf LB-Platten mit 100 µg/ml Ampicillin plattiert und über Nacht bei 37 °C inkubiert.

Die Kolonien wurden auf Nitrocellulose-Filter übertragen, Replikaplattiert, mit 0,5 N NaOH/1,5 M NaCl lysiert und die denaturierte DNA durch 2-stündiges Backen bei 80 °C fest an den Filter gebunden.
Die Filter wurden in 6 x SET-Puffer (1 x SET = 0,15 M NaCl, 15mM Tris/HCL pH 7,4, 1 mM EDTA), 0,1 % SDS und 5 x Denhardt's Lösung (100 x Denhardt = 1 g Ficoll, 1 g Polyvinylpyrrolidon, 1 g BSA pro 50 ml) für 4 h, bei 68 °C vorhybridisiert. Mit Hilfe eines DNA-Synthesizers wurden drei Oligonukleotid-Sonden, die jeweils 17 Basen der tPA-DNA umfassen, hergestellt. Sie bestehen aus folgenden Sequenzen:

5'-TGCAGATCACTTGGTAA-3'
5'-CCAGGCCCAGTGCCTGG-3'
5'-TCCAGTCCGGCAGCTGC-3'

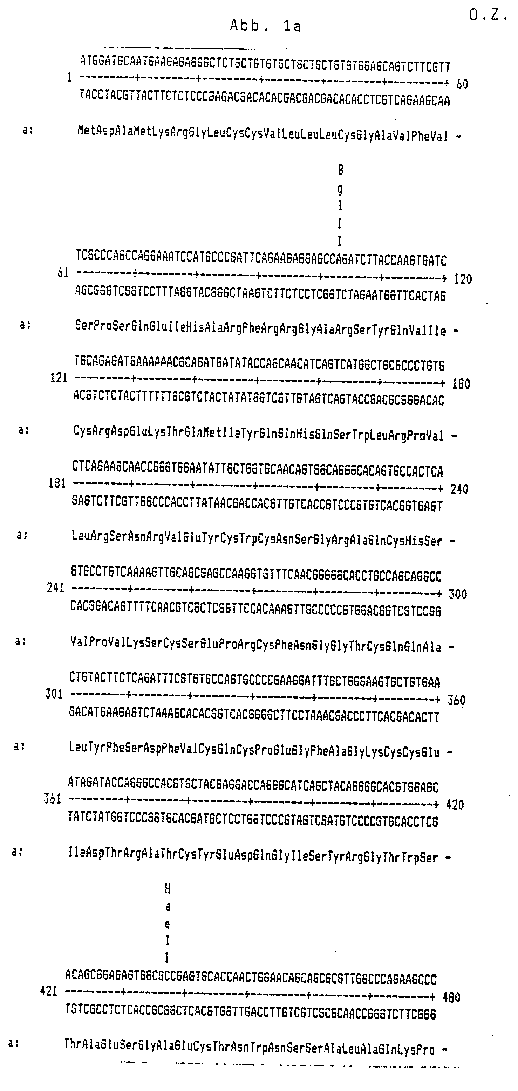

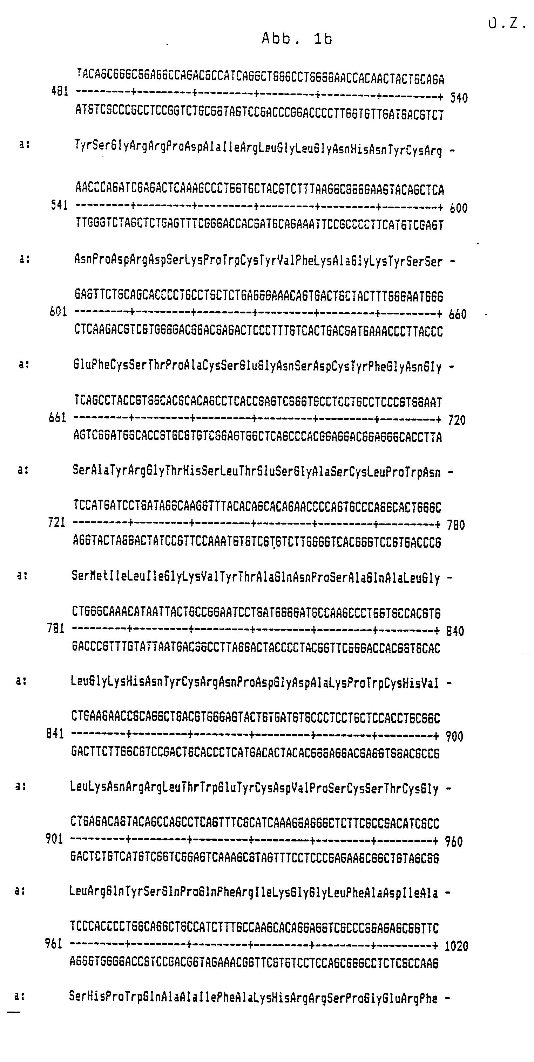

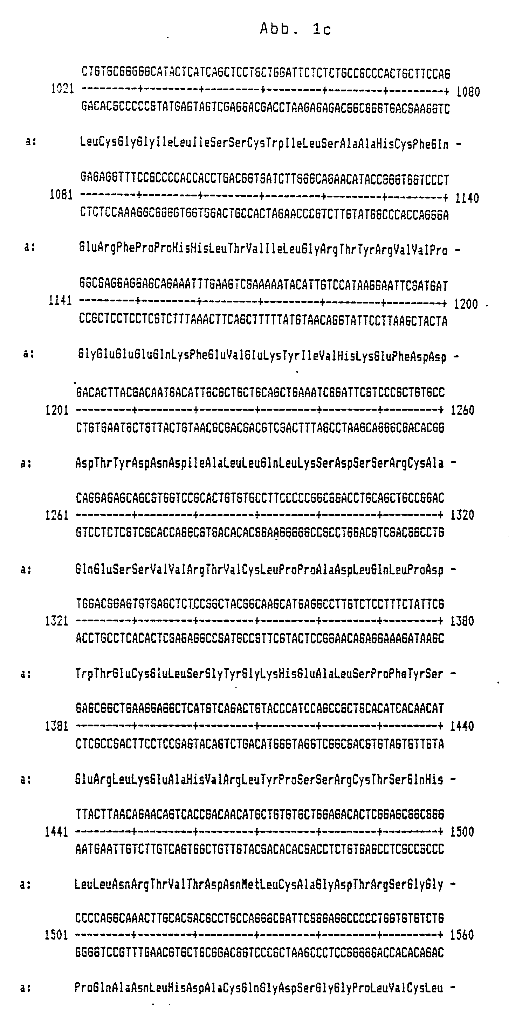

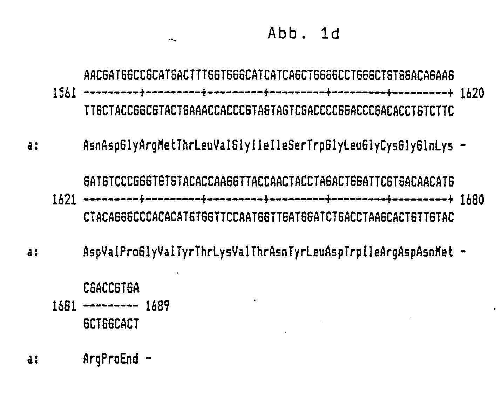

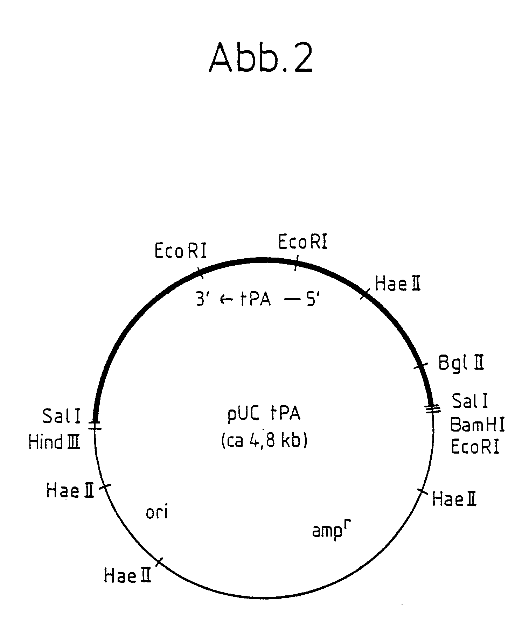

Diese Sonden wurden am 5'-Ende mit $\gamma$-32P-ATP markiert. Sie wurden dann mit den vorhybridisierten Filtern in einer Lösung, die 6 x SET, 0,1 % SDS, 5 x Denhardt's und 10 % Dextransulfat enthält, über Nacht bei 42°C unter leichtem Schütteln inkubiert. Die Filter werden danach mehrfach in 6 x SET/0,1 % SDS bei 42°C gewaschen, angetrocknet und einem Röntgenfilm exponiert. Klone, die beim "Screening" eine radioaktive Antwort gaben, wurden isoliert und weitergezüchtet. Ein Klon, im folgenden pUCtPA genannt, enthält ein etwa 2,1 kb großes Insert, das die codierende Region, sowie 5'- und 3'-nicht-codierende Bereiche enthält (Abb. 2). Plasmid-DNA von pUCtPA wurde durch Lysozym-Aufschluß und SDS-Alkali-Behandlung der Bakterienkultur sowie anschließende CsCl-Gradientenzentrifugation präpariert.

Beispiel 2

Insertion von Oligonukleotiden in die tPA-DNA

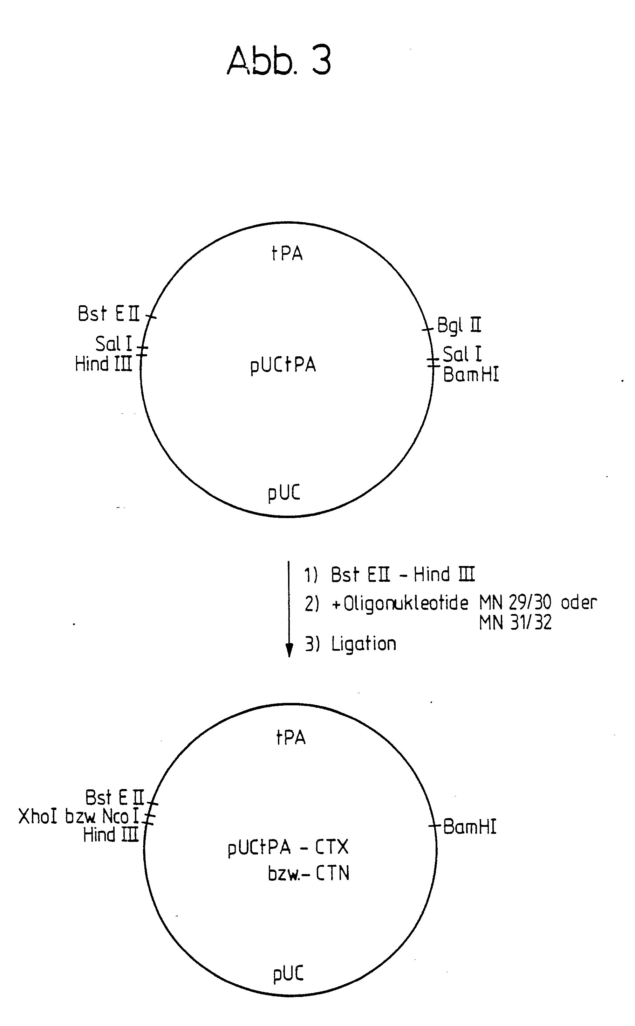

Ausgangspunkt war das Plasmid pUCtPA. Es wurde präparativ mit BStEII und HindIII geschnitten (Abb. 3). Anschließend erfolgte eine Behandlung mit alkalischer Phosphatase.

Mit Hilfe eines DNA-Synthesizers wurden folgende Oligonukleotide hergestellt und aufgereinigt:
MN 29 5'-GTTACCAACTACCTAGACTGGATTCGTGACAACCTCGAGTGAGTCGACA-3'
MN 30 5'-AGCTTGTCGACTCACTCGAGGTTGTCACGAATCCAGTCTAGGTAGTTG-3'
MN 31 5'-GTTACCAACTACCTAGACTGGATTCGTGACAACCCATGGTGAGTCGACA-3'
NM 32 5'-AGCTTGTCGACTCACCATGGGTTGTCACGAATCCAGTCTAGGTAGTTG-3'

Die Oligonukleotide wurden in einer "annealing"-Reaktion miteinander hybridisiert und gleichzeitig durch Behandlung mit Polynukleotidkinase phosphoryliert.

50 ng der vorbereiteten Oligonukleotide wurden dann mit 400 ng der Vektor-DNA ligiert. Mit dem Gemisch wurden anschließend kompetente HB101-Zellen transformiert, welche auf Ampicillin-haltigem Medium auf Plasmid-Gegenwart selektiert werden. Aus 2 ml-über-Nacht-Kulturen wurden als sog. "Minilysate" kleine Mengen Plasmid-DNA freigesetzt und mit Restriktionsenzymen folgende Spaltungen durchgeführt:

bei der Konstruktion mit MN 29/30:

BamHI + XhoI (Ansatz 1),
EcoRI + XhoI (Ansatz 2) oder
HaeII + SmaI (Ansatz 3)

bei der Konstruktion mit MN 31/32:

BamHI + NcoI (Ansatz 1),
EcoRI + NcoI (Ansatz 2) oder
HaeII + SmaI (Ansatz 3).

Die erhaltenen Fragmente wurden durch Gelelektrophorese aufgetrennt. Das Vorliegen der korrekten und einfachen Oligonukleotid-Insertion zeigte sich im Vergleich zur Ausgangs-DNA durch teilweise veränderte Fragmentgrößen; auch die Orientierung ließ sich auf diese Weise feststellen. Die abschließende Untersuchung war die Analyse der DNA-Sequenz, der Oligonukleotid-Insertion und der angrenzenden Bereiche (nach Sanger et al. 1977, Proc. Natl. Acad. Sci. USA 74, 5463-67). Große Mengen Plasmid-DNA eines Kandidaten mit korrekter und einfacher Insertion wurde durch Aufschluß von 1 l Über-Nacht-Kultur des betreffenden Stammes gewonnen. Die DNA ("pUCtPA-CTX" mit MN 29/30 bzw. "pUCtPA-CTN" mit MN 31/32) wurde zweimal über CsCl-Gradienten gereinigt und ausgiebig gegen TE-Puffer dialysiert.

Beispiel 3a

Insertion von Oligonukleotiden in die tPA-DNA

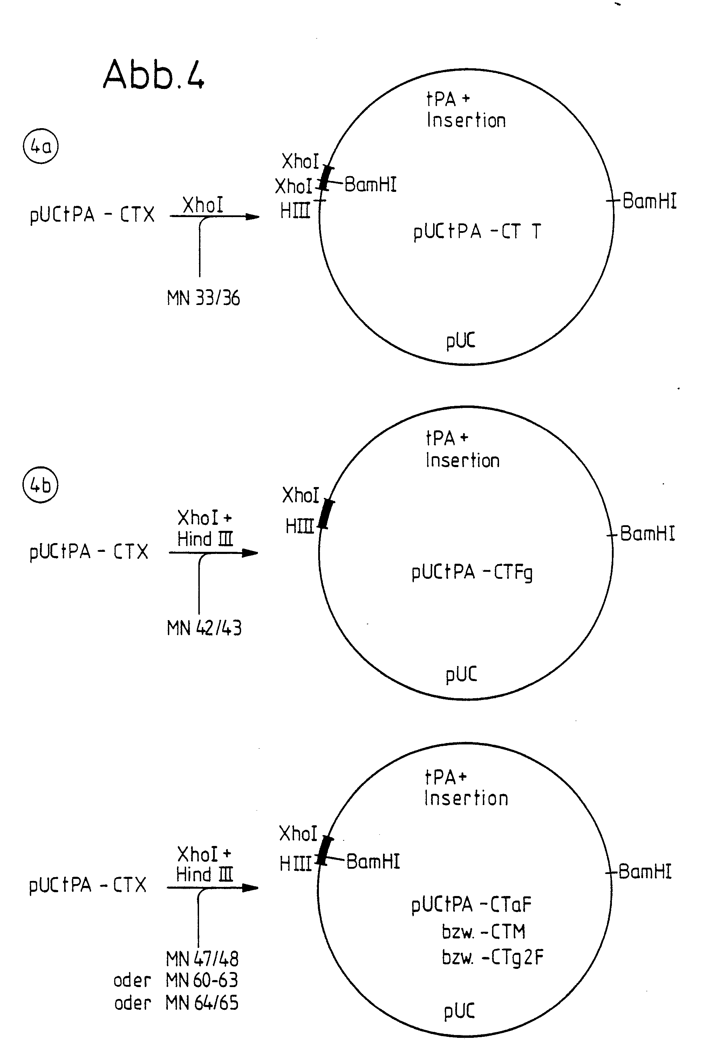

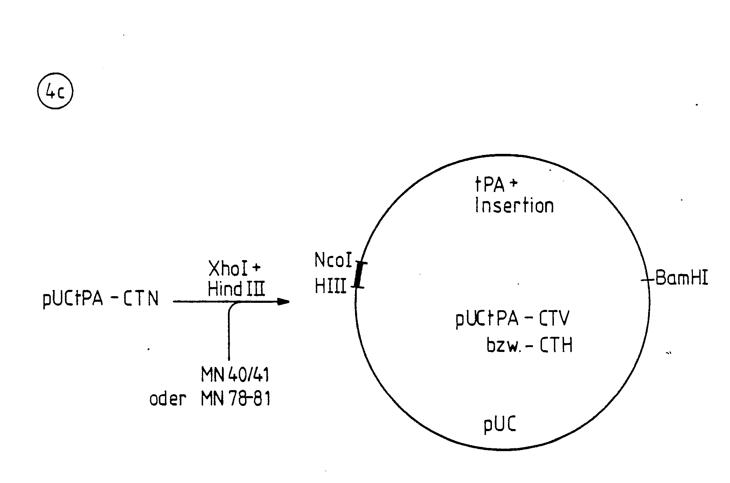

Ausgangspunkt war das Plasmid pUCtPA-CTX. Es wurde präparativ mit dem Restriktionsenzym XhoI gespalten (Abb. 4). Anschließend erfolgte eine Behandlung mit alkalischer Phosphatase.

Mit Hilfe eines DNA-Synthesizers wurden folgende Oligonukleotide hergestellt und aufgereinigt:
MN33 5'-TCGAGAACCCTGATCAGGCCGA-CAGCGATGGGGACGGCAGAGGCGACGCCTGCG-ATGACATCGA-3'
MN34 5'-CGTCGCCTCTGCCGTCCCCATCGCTGTCGGCC-TGATCAGGGTTC-3'
MN35 5'-CAACGATGGGATCCCAGACGAGGACAATTGTCC-TC-3'
MN36 5'-TCGAGAGGA-CAATTGTCCTCGTCTGGGATCCCATCGTTGTCG-ATGTCATCGCAGG-3'

Die Oligonukleotide wurden in einer "annealing"-Reaktion miteinander hybridisiert und gleichzeitig durch Behandlung mit Polynukleotidkinase phosphoryliert.

100 ng der vorbereiteten Oligonukleotide wurden dann mit 400 ng der Vektor-DNA ligiert. Mit dem Gemisch wurden anschließend kompetente HB101-Zellen transformiert, welche auf Ampicillin-haltigem Medium auf Plasmid-Gegenwart selektiert wurden. Aus 2 ml-über-Nacht-Kulturen wurden als sog. "Minilysate" kleine Mengen Plasmid-DNA freigesetzt und mit folgenden Restriktionsenzymen gespalten:

    1) BamHI
    2) BamHI-HaeII
    3) BstEII-HaeII

Die erhaltenen Fragmente wurden gelelektrophoretisch aufgetrennt. Das Vorliegen der korrekten und einfachen Oligonukleotid-Insertion zeigte sich im Vergleich zur Ausgangs-DNA durch teilweise veränderte Fragmentgrößen; auch die Orientierung ließ sich auf diese Weise feststellen. Die abschließende Untersuchung war die Analyse der DNA-Sequenz analog Beispiel 2. Große Mengen Plasmid-DNA jeweils eines Kandidaten mit korrekter und einfacher Insertion wurden durch Aufschluß von 1 l über-Nacht-Kultur des betreffenden Stammes gewonnen. Die DNA ("pUCtPA-CT-T") wurde zweimal über CsCl-Gradienten gereinigt und ausgiebig gegen TE-Puffer dialysiert.

Beispiel 3b

Insertion von Oligonukleotiden in die tPA-DNA

Ausgangspunkt war das Plasmid pUCtPA-CTX. Es wurde präparativ mit den Restriktionsenzymen XhoI + HindIII gespalten und die ca. 4,5 kb große Bande gelelektrophoretisch isoliert (Abb. 4).

Mit Hilfe eines DNA-Synthesizers wurden folgende Oligonukleotide hergestellt und aufgereinigt:
MN42    5'-TCGAGACCAGATGGGTGAGCATGAA-GAAAACCACAATGAAGTGAGTCGACA-3'
MN43    5'-AGCTTGTCGACTCACTT-CATTGTGGTTTTCTTCATGCTCACCCATCTGGT-C-3'
MN47  5'-TCGAGAAACGACTGGAGGTGGACATT-GATATTAAGATCCGATCTTGAGTCGACGGATCC-A-3'
MN48    5'-AGCTTGGATCCGTCGACTCAA-GATCGGATCTTAATATCAATGTCCACCTCCAGT-CGTTTC-3'
MN  60   5'-TCGAGGGAATTGGAGCAGTACT-GAAGGTATTAACCACAGGATTGCCCGCCCTCAT-AAGTTGGA-3'
MN    61    5'-CAATCCTGTGGTTAATACCTTCAGTACTGCTCCAATTCCC-3'
MN    62    5'-TTAAACGTAAGAGGCAACAGGGTTGAGTCGACGGATCCA-3'
MN  63   5'-AGCTTGGATCCGTCGACT-CAACCCTGTTGCCTCTTACGTTTAATCCAACTTA-

TGAGGGCGGG-3'
MN 64 5'-TCGAGCACCACTTGGGGGGAGCCAAACAGGCTCGAGACGTTTGAGTCGACGGATCCA-3'
MN  65   5'-AGCTTGGATCCGTCGACTCAA-ACGTCTCGAGCCTGTTTGGCTCCCCCCAAGTGG-TGC-3'

Die Oligonukleotide MN 42 + 43 (Ansatz a), MN 47 + 48 (Ansatz b), MN 60-63 (Ansatz c) sowie MN 64 + 65 (Ansatz d) wurden jeweils in einer "annealing"-Reaktion miteinander hybridisiert.

Jeweils 200 ng der vorbereiteten, nicht phosphorylierten Oligonukleotide wurden dann mit je 100 ng der Vektor-DNA ligiert. Mit dem Gemisch wurden anschließend kompetente HB101-Zellen transformiert, welche auf Ampicillin-haltigem Medium auf Plasmid-Gegenwart selektiert wurden. Aus 2 ml-über-Nacht-Kulturen wurden als sog. "Minilysate" kleine Mengen Plasmid-DNA freigesetzt und mit folgenden Restriktionsenzymen gespalten:

    Ansatz a:
SacI + HindIII
    Ansatz b:
    1) SacI + HindIII
    2) BamHI
    Ansatz c:
    1) BamHI
    2) ScaI
    3) SacI + SalI
    Ansatz d:
    1) BamHI
    2) BglI

Die erhaltenen Fragmente wurden gelelektrophoretisch aufgetrennt. Das Vorliegen der korrekten und einfachen Oligonukleotid-Insertion zeigte sich im Vergleich zur Ausgangs-DNA durch teilweise veränderte Fragmentgrößen; auch die Orientierung ließ sich auf diese Weise feststellen. Die abschließende Untersuchung war die Analyse der DNA-Sequenz analog Beispiel 2. Große Mengen Plasmid-DNA jeweils eines Kandidaten mit korrekter und einfacher Insertion wurden durch Aufschluß von 1 l über-Nacht-Kultur des betreffenden Stammes gewonnen. Die DNA ("pUCtPA-CTFg" (a), "pUCtPA-CTaF" (b), "pUCtPA-CTM" (c), "pUCtPA-CTg2F" (d)) wurde zweimal über CsCl-Gradienten gereinigt und ausgiebig gegen TE-Puffer dialysiert.

Beispiel 3c

Insertion von Oligonukleotiden in die tPA-DNA

Ausgangspunkt war das Plasmid pUCtPA-CTN. Es wurde präparativ mit den Restriktionsenzymen XhoI + HindIII gespalten (Abb. 4).

Mit Hilfe eines DNA-Synthesizers wurden folgende Oligonukleotide hergestellt und aufgereinigt:

MN40       5'-CATGGGGCGAGTGCTGTGGCA-GATGCCTGCCCAGCTGAGTCGACA-3'

MN41                5'-AGCTTGTCGACT-CAGCTGGGCAGGCATCTGCCACAGCACTCGCC-C-3'

MN78       5'-CATGGAGAGGCGACACCCCGAA-ACCGCAGTCTCACAACGACGGCGACTTCGAAGA-AATC-3'

MN79                      5'-CGTCGTTGTGAGACTGCGGTTTCGGGGTGTCG-CCTCTC-3'

MN80                      5'-CCGGAAGAATACCTGCAGTAATGATCTAGAGTC-GACA-3'

MN81       5'-AGCTTGTCGACTCTAGATCAT-TACTGCAGGTATTCTTCCGGGATTTCTTCGAAG-TCGC-3'

Die Oligonukleotide MN 40 + 41 (Ansatz a) sowie MN 78-81 (Ansatz b) wurden jeweils in einer "annealing"-Reaktion miteinander hybridisiert.

150 ng der vorbereiteten, nicht phosphorylierten Oligonukleotide wurden dann mit 400 ng der Vektor-DNA ligiert. Mit dem Gemisch wurden anschließend kompetente HB101-Zellen transformiert, welche auf Ampicillin-haltigem Medium auf Plasmid-Gegenwart selektiert wurden. Aus 2 ml-über-Nacht-Kulturen wurden als sog. "Minilysate" kleine Mengen Plasmid-DNA freigesetzt und mit folgenden Restriktionsenzymen gespalten:

      Ansatz a:

SacI + HindIII

      Ansatz b:

        1) SacI + HindIII

        2) SacI + HaeII

        3) PstI

Die erhaltenen Fragmente wurden gelelektrophoretisch aufgetrennt. Das Vorliegen der korrekten und einfachen Oligonukleotid-Insertion zeigte sich im Vergleich zur Ausgangs-DNA durch teilweise veränderte Fragmentgrößen; auch die Orientierung ließ sich auf diese Weise feststellen. Die abschließende Untersuchung war die Analyse der DNA-Sequenz analog Beispiel 2. Große Mengen Plasmid-DNA jeweils eines Kandidaten mit korrekter und einfacher Insertion wurden durch Aufschluß von 1 l über-Nacht-Kultur des betreffenden Stammes gewonnen. Die DNA ("pUCtPA-CTV" (a), "pUCtPA-CTH (b)) wurde zweimal über CsCl-Gradienten gereinigt und ausgiebig gegen TE-Puffer dialysiert.

Beispiel 4

Konstruktion von Vektoren für die Expression der modifizierten tPA-DNA

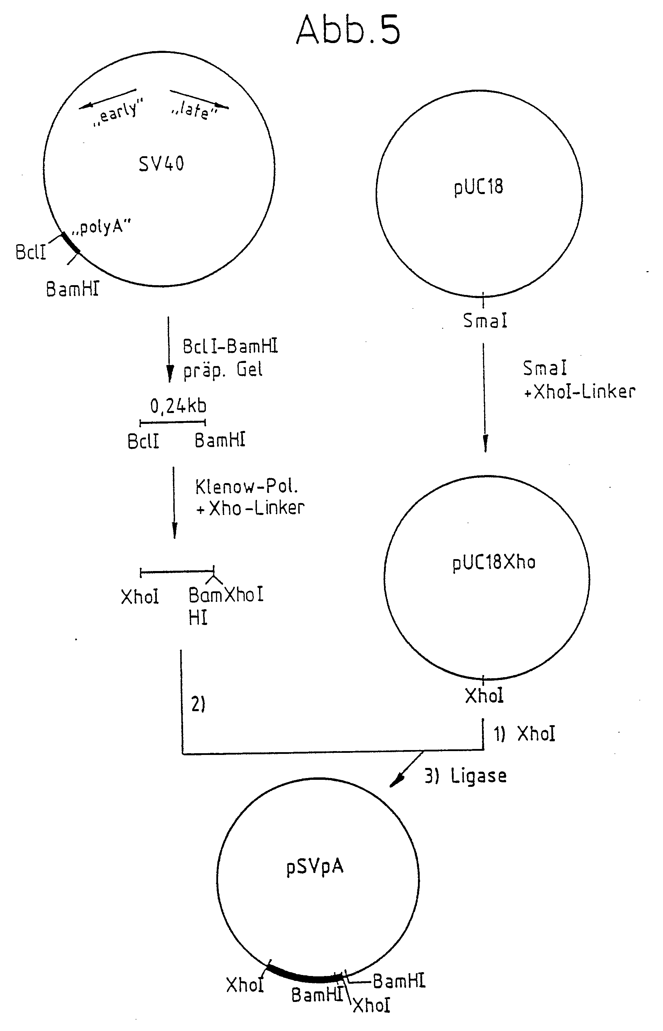

DNA des Affenvirus SV40 wurde mit den Restriktionsenzymen BamHI und BclI geschnitten und das 0,24 kb-Fragment gelelektrophoretisch präpariert (Abb. 5). Die Enden wurden in Gegenwart der vier Desoxynukleotidtriphosphate dATP, dCTP, dGTP und dTTP mit dem Klenow-Fragment der DNA-PolymeraseI aufgefüllt. Anschließend wurden XhoI-Linker anligiert.

Parallel wurde der kommerziell erhältliche Vektor pUC18 mit dem Enzym SmaI linearisiert. Dann wurden ebenfalls XhoI-Linker angesetzt. DNA dieses Vektors ("pUC18Xho") wurde mit XhoI linearisiert, mit alkalischer Phosphatase behandelt und mit dem 0,24 kb XhoI-SV40-Fragment (s. o.) ligiert. Es entstand pSVpA.

pSVpA-DNA wurde präparativ mit XhoI gespalten und wie oben mit Klenow-Polymerase in Gegenwart der vier dNTPs inkubiert. Das 0,24 kb-Fragment wurde Gel-isoliert.

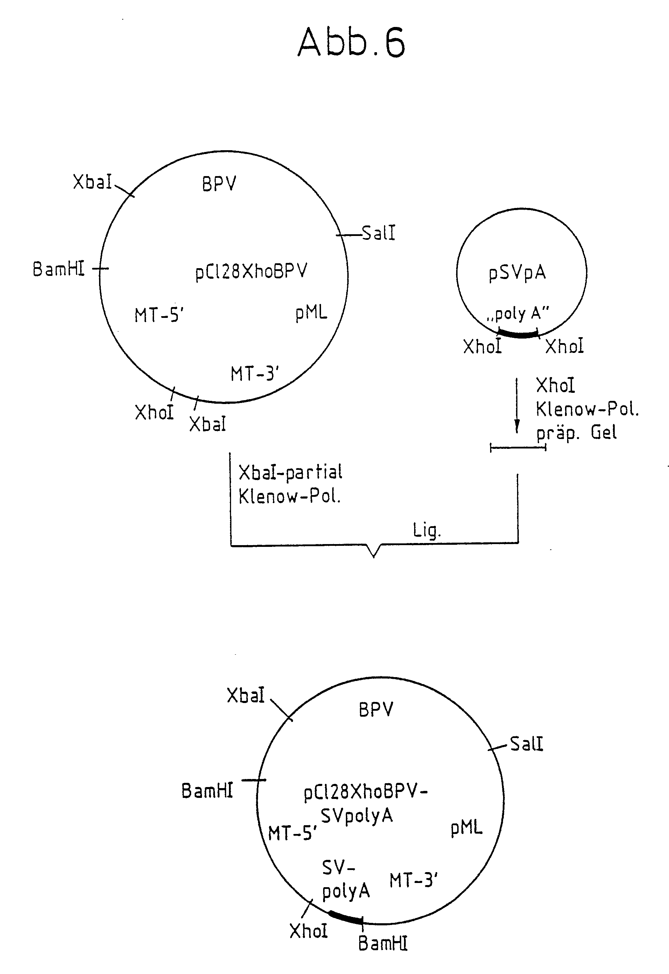

Gleichzeitig wurde der Eukaryonten-Expressionsvektor CL28XhoBPV, entstanden durch Ligation von CL28X und pB2-2 (nach Reddy et al. 1987, DNA 6, 461-72), partiell mit dem Restriktionsenzym XbaI geschnitten, d. h. es wurde zeitlich derart limitiert inkubiert, daß Moleküle entstanden, die nur an einer der beiden XbaI-Erkennungssequenzen gespalten, also linearisiert sind (Abb. 6). Der Ansatz wurde dann wie beschrieben mit Klenow-Polymerase und dNTPs umgesetzt. Die linearen Moleküle wurden anschließend durch Gelelektrophorese isoliert.

Es erfolgte dann die Ligation der linearen pCL28XhoBPV-Fragmente mit dem vorbehandelten 0,24 kb-Fragment aus SV40. Nach Transformation und Screening von Minilysaten wurde ein Klon isoliert, der das SV40-Fragment in der etwa 0,15 kB 3'-wärts der XhoI-Stelle gelegenen vormaligen XbaI-Stelle trug; diese DNA ("pCL28XhoBPV-SVpolyA") trug die SV40-Transkriptionsstopsignale der "frühen" Gene.

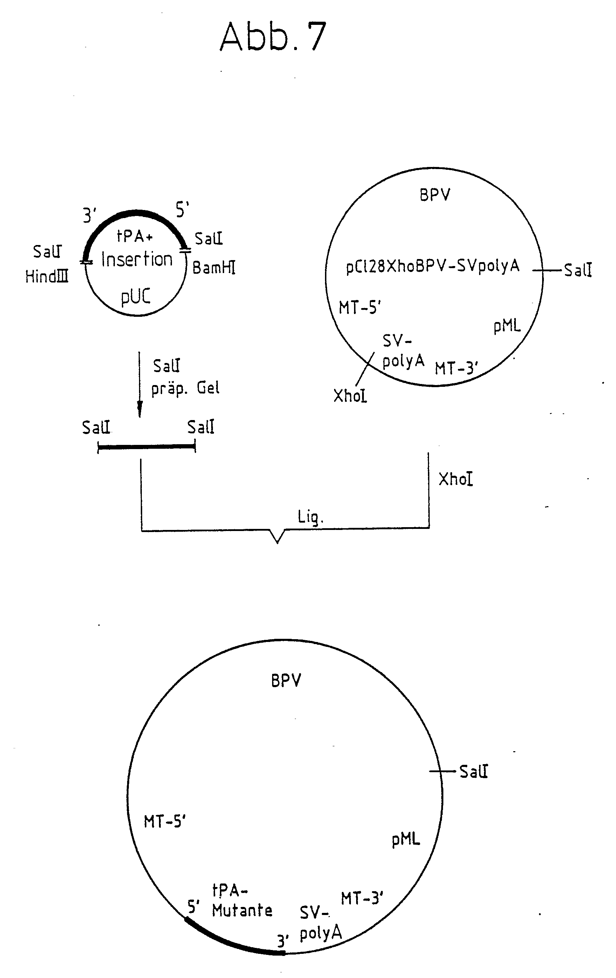

Plasmid-DNA von pCL28XhoBPV-SVpolyA wurde mit dem Restriktionsenzym XhoI linearisiert und mit alkalischer Phosphatase behandelt. Gleichzeitig wurde pUCtPA-CTX, -CTN, -CTT, -CTFg, -CTaF, -CTM, -CTg2F, -CTV, -CTH (aus den Beispielen 2 und 3) mit dem Enzym SalI gespalten und das ca. 1,8 kb große Insert präpariert (Abb. 7). Beide Fragmente wurden mittels T4-Ligase miteinander verbunden. Nach Transformation und Minilysaten wurde ein Klon isoliert, der die mutierte tPA-DNA einfach und in der korrekten Orientierung enthielt: pCL28BPV-tPA-CTX, -CTN, -CTT, -CTFg, -CTaF, -CTM, -CTg2F, -CTV, -CTH.

Beispiel 5

Transfektion und Etablierung von Zellinien

C127I Zellen (J. Virol. 26 (1978) 292; ATCC catalogue of cell lines and hybridomas 5th edition, 1985, p142) wurden mit BPV-Expressionsplasmiden transfiziert mit der Calciumphosphat-Copräzipitationsmethode (Virology 52 (1973)/ 456, DNA cloning; volume II; ed. D.M.Glover IRL Press, Seiten 143ff und 213 (1985)). $5 \times 10^5$ C127I-Zellen wurden in DMEM (Dulbecco's Modified Eagles Medium) + 10 % FCS (Foetales Kalbsserum) in (60 mm Petrischalen eingesät. Am nächsten Tag wurde das Medium gewechselt auf MEM (Modified Eagles Medium) mit 25mM Hepes + 10 % FCS. Mit 10 μg CsCl-gereinigter Plasmid DNA wurde ein Ca-Phosphat-Copräzipitat gebildet, welches vorsichtig auf die C127I-Zellen aufgebracht wurde. Die Zellen wurden 4 h bei 37°C; 7 % $CO_2$ inkubiert. Durch eine anschließende Glycerin-Schock-Behandlung wurde die Effizienz der Transfektion erheblich gesteigert. Hierzu wurde 4 h nach Aufbringen des Präzipitates das Medium von den Zellen abgezogen. Die Zellen wurden 3 min mit je 2 ml 15 % Glycerin/HBS (DNA cloning Vol. II, Seite 152) pro 60 mm Petrischale bei Raumtemperatur inkubiert. Die Glycerin/HBS-Lösung wurde abgezogen, der Zellrasen mit 3 ml DMEM + 10 % FCS gewaschen. Die Zellen wurden mit DMEM + 10 % FCS bei 37°C; 7 % $CO_2$ inkubiert. Dreimal in der Woche wurde das DMEM + 10 % FCS abgezogen und durch frisches ersetzt. Nach 2-3 Wochen waren transfizierte Zellen, die das BPV-Genom enthalten, als Ansammlungen transformierter Zellen, sogenannte Foci, zu erkennen. Foci, die die oben beschriebenen tPA-Muteine exprimieren, wurden durch einen Casein-Agar-Overlay (siehe unten) identifiziert. Nach 2-3 h Inkubation bei 37°C; 7 % $CO_2$ waren im trüben Casein-Agar Lysehöfe an den Stellen zu erkennen, an denen sich tPA-exprimierende Zellen befinden. Nach Entfernen des Casein-Agar wurden die sich an diesen Stellen befindenden Zellen nach der "cloning-cylinder"-Methode isoliert (DNA cloning Vol. II, S. 220). Größere Mengen an Zellen wurden in Spinnerflaschen produziert. Hierzu wurden 1-Liter-Spinnerflaschen mit 6g/l ®Cytodex III und $2 \times 10^8$ Zellen in einem Volumen von 1000 ml DMEM + 10 % FCS angeimpft. Das Verfahren der Mikrocarrierkultur ist beschrieben in "Microcarrier cell culture; principles and methods (Pharmacia Fine Chemicals; D-7800 Freiburg). Nach Erreichen der Konfluenz (ca. 1,5 x $10^6$ Zellen/ml) wurden die Zellen in serumfreien DMEM gehalten.

Für den oben erwähnten Agar Overlay Test werden folgende Lösungen benötigt:

Overlay-Agarose

1) 8 % Magermilchlösung in $H_2O$ werden 30 min bei 100°C gekocht und anschließend im 37°C Wasserbad abgekühlt.

2) Eine 2 %ige Lösung von Low-melting Agarose in PBS wird nach Autoklavieren im 37°C Wasserbad abgekühlt. Anschließend wird im Verhältnis 1:1 doppelt konzentriertes DMEM zugegeben.

3) 0,64 mg Plasminogen wird in 1 ml $H_2O$ gelöst. Durch Zusammenpipettieren von 16 ml Lösung 2, 4 ml Lösung 1 und 0,4 ml Plasminogen wird die Overlay Agarose hergestellt. Diese wird nach Mischen im 37°C Wasserbad gehalten.

Zur Durchführung des Tests werden die Zellen in 60 mm Petrischalen zweimal mit serumfreiem Medium gewaschen. Anschließend werden je 2 ml "Overlay-Agarose" vorsichtig in die Petrischalen pipettiert. Die Petrischalen werden zum Abkühlen und Erstarren der Agarose bei Raumtemperatur stehen gelassen. Anschließend wird 2-3h im Brutschrank bei 37°C unter Zugabe von 7 % $CO_2$ inkubiert und die Größe und Anzahl der Lysehöfe bestimmt.

Beispiel 6

Isolierung eines neuen Polypeptids

Aus dem gemäß Beispiel 5 erhaltenen serumfreien Zellkulturüberstand wurde das tPA-Mutein nach Sterilfiltration und Zugabe von 0,01 % ®Tween 80 durch eine Affinitätschromatographie an Erythrina-Trypsin-Inhibitor-Sepharose (ETI-Sepharose, 1 cm x 3 cm) isoliert und durch eine anschließende Lysin-Sepharose Chromatographie gereinigt. Zur Herstellung der (ETI-Affinitätsmatrix wurden 5 mg ETI pro ml an CNBr-aktivierte Sepharose 48 gekoppelt. Eine detaillierte Beschreibung der Vorgehensweise ist der Vorschrift des Herstellers (Pharmacia, Freiburg, FRG) zu entnehmen. Das Gelmaterial wurde vor dem Auftragen des Zellkulturüberstands mit 20 mM Na-phosphat, 0,15 M NaCl, 0,01 % ®Tween 80, pH 7,0 äquilibriert. Nach dem Auftragen wurde das Gelmaterial mit demselben Puffer behandelt, um unspezifisch gebundenes Material zu entfernen. Die Desorption des spezifisch gebundenen Muteins erfolgte anschließend durch Elution mit 0,1 M Glycin, 0,1 M Arginin/HCl, 0,01 % ®Tween 80. pH 3,0. Das Eluat wurde vereinigt und mit 0,1 M Natronlauge auf pH 7,0 eingestellt.

Die Reinigung der Muteine erfolgte dann durch eine Lysin-Sepharose-Chromatographie. Diese wurde mit der kommerziell erhältlichen Gelmatrix von Pharmacia durchgeführt. Dabei wurde wie folgt Verfahren: Das Gelmaterial wurde mit 20 mM Na-phosphat, 250 mM NaCl, 0,01 % ®Tween 80 pH 7,0 äquilibriert. Vor dem Auftrag wurde die Wertfraktion der ETI-Sepharose 1:5 (v/v) mit dem Äquilibrierungspuffer der Lysin-Sepharose-Säule verdünnt. Nach dem Wegwaschen des nicht gebundenen Materials mit 20 mM Na-phosphat, 0,01 % ®Tween 80 pH 6,5 erfolgte die Elution des Muteins mit 20 mM Na-phosphat, 0,4 M Arginin, 0,01 % ®Tween 80 pH 6,5. Mit der Dialyse der Wertfraktion gegen 20 mM Na-phosphat, 0,1 M Arginin, 0,15 M NaCl, 0,01 % ®Tween 80 pH 5,0 ist die Reinigung des Muteins abgeschlossen.

Beispiel 7

Charakterisierung des Oligosaccharid-Anteils

5 - 10 µg des gemäß Beispiel 6 erhaltenen Muteins wurden durch SDS-Gelelektrophorese aufgetrennt und anschließend auf Nitrozellulose-Membranen transferiert. Nach dem Absättigen mit PBS-Puffer (2 mM Phosphat, 150 mM NaCl, pH 7,4), der 0,1 % ®Tween 20, pH 7,4 enthielt, wurde die Membran 2 h mit an Peroxidase gekoppeltem Lektin von Griffonia Simplicifolia inkubiert. Nach dem Entfernen ungebundenen Materials mit Hilfe von TBS-Puffer (10 mM Tris, 150 mM NaCl, pH 7,4) wurde die Nitrozellulose 5 - 10 min in 50 mM Tris, 150 mM NaCl, 0,02 % $H_2O_2$ und 0,5 mg/ml 4-Chlor-1-naphthol inkubiert. Positive Reaktionen wurden durch Blaufärbung der Proteinbande innerhalb von 5 min sichtbar. Die Reaktion wurde dann durch Transferieren der Nitrozellulose-Membran in Wasser gestoppt.

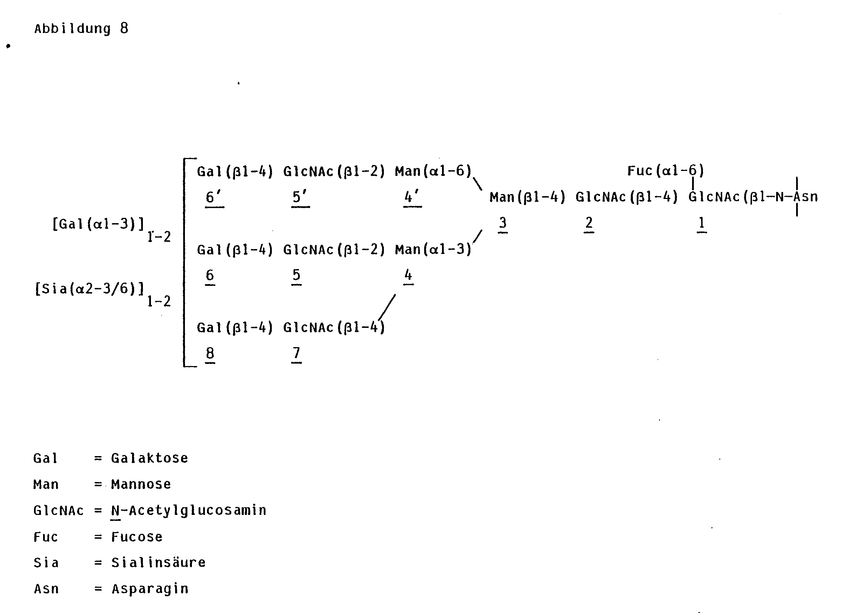

Die Oligosaccharid-Reste enthalten α-gebundene Galaktose-Reste, die gemäß Abb. 8 mit ß-gebundenen subterminalen Galaktose-Resten verknüpft sind. Dabei sind die α-Galaktose-Reste bevorzugt an der Galaktose 6' (Numerierung der Zuckerreste siehe Abb. 8) lokalisiert. Die anderen subterminalen Galaktose-Reste sind entweder durch Sialinsäure oder weitere α-gebundene Galaktose substituiert.

Beispiel 8

Bestimmung der in-vitro-Thrombolyse im "Chandler Loop"

In einem Polyethylenschlauch (Länge 27,5 cm, Innendurchmesser 4 mm) wurden 2 ml citriertes Vollblut mit radioaktivem $^{125}$-Jod-Fibrinogen gemischt. Das Citratblut wurde durch Zugabe von 200 µl 0,25 mol/l $CaCl_2$-Lösung rekalzifiziert. Auf einer Drehtrommel wurde der Ring bei einem Neigungswinkel von 23° C und einer Temperatur von 37° C rotiert.

Nach 30 min wurde der Thrombus entnommen, gewogen und der Radioaktivitätsgehalt gemessen (Ziel: 90 000 - 110 000 cpm/clot).

In einen Polyethylenschlauch wurden 2 ml autologes Citratplasma und je ein markierter Thrombus gegeben. Dann wurden die Prüfsubstanzen bzw. die Kontrollösung (Vehikel) in den Schlauch pipettiert. Vor (0 min - Kontrolle) und 30 min, 60 min und 180 min nach Substanz- bzw. Vehikel-Applikation wurden 200 µl Plasma entnommen und deren Radioaktivitätsgehalt bestimmt. Während des ganzen Versuches rotierte die Drehtrommel mit 12 Umdrehungen pro min.

Nach 180 min wurde die Restradioaktivität und das Restgewicht des Thrombus bestimmt.

Die Thrombolyse wurde berechnet als die Differenz von Gewicht bzw. Radioaktivitätsgehalt zu den Zeitpunkten 0 min und 3 h, ausgedrückt in Prozent vom Ausgangswert:

$$\text{Thrombolyse} = \frac{T_0 - T_{3h}}{T_0} \times 100$$

Die in den Beispielen dargestellten tPA-Muteine zeigten in diesem Versuch gegenüber nichtverändertem tPA eine bessere Thrombolyse.

Ansprüche

1. Polypeptide der Formel
tPA$_{1-524}$-B,
worin
tPA$_{1-524}$ die Aminosäuren 1-524 des humanen, gereiften Gewebe-Plasminogenaktivators und
B eine der Aminosäuresequenzen
- LE
- PW
-       LENPDQADSDGDGRGDACDDIDNDGIPDEDNCPG
- LETRWVSMKKTTMK
- PWGECCGRCLPS
- LEKRLEVDIDIKIRS
- LEGIGAVLKVLTTGLPALISWIKRKRQQG
- LEHHLGGAKQARDV
- PWRGDTPKPQSHNDGDFEEIPEEYLQ
bedeuten, sowie deren Allelvarianten oder Derivate mit am N- und/oder C-Terminus von B um ein bis sechs Aminosäuren verkürzten oder verlängerten Sequenzen.

2. DNA-Sequenzen, die für die Polypeptide gemäß Anspruch 1 kodieren.

3. Vektoren, die Gensequenzen enthalten, die für die Polypeptide gemäß Anspruch 1 kodieren.

4. Gentechnologisches Verfahren zur Herstellung von Peptidsequenzen nach Anspruch 1, dadurch gekennzeichnet, daß man in einem Wirtsorganismus ein Gen zur Expression bringt, das für die Peptidsequenzen nach Anspruch 1 kodiert und das Protein isoliert.

9

Abb. 1a

```
    ATGGATGCAATGAAGAGAGGGCTCTGCTGTGTGCTGCTGCTGTGTGGAGCAGTCTTCGTT
1   ---------+---------+---------+---------+---------+---------+ 60
    TACCTACGTTACTTCTCTCCCGAGACGACACACGACGACGACACACCTCGTCAGAAGCAA

a:  MetAspAlaMetLysArgGlyLeuCysCysValLeuLeuLeuCysGlyAlaValPheVal -
```

```
                                B
                                g
                                l
                                I
                                I

    TCGCCCAGCCAGGAAATCCATGCCCGATTCAGAAGAGGAGCCAGATCTTACCAAGTGATC
61  ---------+---------+---------+---------+---------+---------+ 120
    AGCGGGTCGGTCCTTTAGGTACGGGCTAAGTCTTCTCCTCGGTCTAGAATGGTTCACTAG

a:  SerProSerGlnGluIleHisAlaArgPheArgArgGlyAlaArgSerTyrGlnValIle -
```

```
    TGCAGAGATGAAAAAACGCAGATGATATACCAGCAACATCAGTCATGGCTGCGCCCTGTG
121 ---------+---------+---------+---------+---------+---------+ 180
    ACGTCTCTACTTTTTTGCGTCTACTATATGGTCGTTGTAGTCAGTACCGACGCGGGACAC

a:  CysArgAspGluLysThrGlnMetIleTyrGlnGlnHisGlnSerTrpLeuArgProVal -
```

```
    CTCAGAAGCAACCGGGTGGAATATTGCTGGTGCAACAGTGGCAGGGCACAGTGCCACTCA
191 ---------+---------+---------+---------+---------+---------+ 240
    GAGTCTTCGTTGGCCCACCTTATAACGACCACGTTGTCACCGTCCCGTGTCACGGTGAGT

a:  LeuArgSerAsnArgValGluTyrCysTrpCysAsnSerGlyArgAlaGlnCysHisSer -
```

```
    GTGCCTGTCAAAAGTTGCAGCGAGCCAAGGTGTTTCAACGGGGGCACCTGCCAGCAGGCC
241 ---------+---------+---------+---------+---------+---------+ 300
    CACGGACAGTTTTCAACGTCGCTCGGTTCCACAAAGTTGCCCCCGTGGACGGTCGTCCGG

a:  ValProValLysSerCysSerGluProArgCysPheAsnGlyGlyThrCysGlnGlnAla -
```

```
    CTGTACTTCTCAGATTTCGTGTGCCAGTGCCCCGAAGGATTTGCTGGGAAGTGCTGTGAA
301 ---------+---------+---------+---------+---------+---------+ 360
    GACATGAAGAGTCTAAAGCACACGGTCACGGGGCTTCCTAAACGACCCTTCACGACACTT

a:  LeuTyrPheSerAspPheValCysGlnCysProGluGlyPheAlaGlyLysCysCysGlu -
```

```
    ATAGATACCAGGGCCACGTGCTACGAGGACCAGGGCATCAGCTACAGGGGCACGTGGAGC
361 ---------+---------+---------+---------+---------+---------+ 420
    TATCTATGGTCCCGGTGCACGATGCTCCTGGTCCCGTAGTCGATGTCCCCGTGCACCTCG

a:  IleAspThrArgAlaThrCysTyrGluAspGlnGlyIleSerTyrArgGlyThrTrpSer -
```

```
                      H
                      a
                      e
                      I
                      I

    ACAGCGGAGAGTGGCGCCGAGTGCACCAACTGGAACAGCAGCGCGTTGGCCCAGAAGCCC
421 ---------+---------+---------+---------+---------+---------+ 480
    TGTCGCCTCTCACCGCGGCTCACGTGGTTGACCTTGTCGTCGCGCAACCGGGTCTTCGGG

a:  ThrAlaGluSerGlyAlaGluCysThrAsnTrpAsnSerSerAlaLeuAlaGlnLysPro -
```

Abb. 1b

```
      TACAGCGGGCGGAGGCCAGACGCCATCAGGCTGGGCCTGGGGAACCACAACTACTGCAGA
  481 ---------+---------+---------+---------+---------+---------+ 540
      ATGTCGCCCGCCTCCGGTCTGCGGTAGTCCGACCCGGACCCCTTGGTGTTGATGACGTCT

   a: TyrSerGlyArgArgProAspAlaIleArgLeuGlyLeuGlyAsnHisAsnTyrCysArg -


      AACCCAGATCGAGACTCAAAGCCCTGGTGCTACGTCTTTAAGGCGGGGAAGTACAGCTCA
  541 ---------+---------+---------+---------+---------+---------+ 600
      TTGGGTCTAGCTCTGAGTTTCGGGACCACGATGCAGAAATTCCGCCCCTTCATGTCGAGT

   a: AsnProAspArgAspSerLysProTrpCysTyrValPheLysAlaGlyLysTyrSerSer -


      GAGTTCTGCAGCACCCCTGCCTGCTCTGAGGGAAACAGTGACTGCTACTTTGGGAATGGG
  601 ---------+---------+---------+---------+---------+---------+ 660
      CTCAAGACGTCGTGGGGACGGACGAGACTCCCTTTGTCACTGACGATGAAACCCTTACCC

   a: GluPheCysSerThrProAlaCysSerGluGlyAsnSerAspCysTyrPheGlyAsnGly -


      TCAGCCTACCGTGGCACGCACAGCCTCACCGAGTCGGGTGCCTCCTGCCTCCCGTGGAAT
  661 ---------+---------+---------+---------+---------+---------+ 720
      AGTCGGATGGCACCGTGCGTGTCGGAGTGGCTCAGCCCACGGAGGACGGAGGGCACCTTA

   a: SerAlaTyrArgGlyThrHisSerLeuThrGluSerGlyAlaSerCysLeuProTrpAsn -


      TCCATGATCCTGATAGGCAAGGTTTACACAGCACAGAACCCCAGTGCCCAGGCACTGGGC
  721 ---------+---------+---------+---------+---------+---------+ 780
      AGGTACTAGGACTATCCGTTCCAAATGTGTCGTGTCTTGGGGTCACGGGTCCGTGACCCG

   a: SerMetIleLeuIleGlyLysValTyrThrAlaGlnAsnProSerAlaGlnAlaLeuGly -


      CTGGGCAAACATAATTACTGCCGGAATCCTGATGGGGATGCCAAGCCCTGGTGCCACGTG
  781 ---------+---------+---------+---------+---------+---------+ 840
      GACCCGTTTGTATTAATGACGGCCTTAGGACTACCCCTACGGTTCGGGACCACGGTGCAC

   a: LeuGlyLysHisAsnTyrCysArgAsnProAspGlyAspAlaLysProTrpCysHisVal -


      CTGAAGAACCGCAGGCTGACGTGGGAGTACTGTGATGTGCCCTCCTGCTCCACCTGCGGC
  841 ---------+---------+---------+---------+---------+---------+ 900
      GACTTCTTGGCGTCCGACTGCACCCTCATGACACTACACGGGAGGACGAGGTGGACGCCG

   a: LeuLysAsnArgArgLeuThrTrpGluTyrCysAspValProSerCysSerThrCysGly -


      CTGAGACAGTACAGCCAGCCTCAGTTTCGCATCAAAGGAGGGGCTCTTCGCCGACATCGCC
  901 ---------+---------+---------+---------+---------+---------+ 960
      GACTCTGTCATGTCGGTCGGAGTCAAAGCGTAGTTTCCTCCCGAGAAGCGGCTGTAGCGG

   a: LeuArgGlnTyrSerGlnProGlnPheArgIleLysGlyGlyLeuPheAlaAspIleAla -


      TCCCACCCCTGGCAGGCTGCCATCTTTGCCAAGCACAGGAGGTCGCCCGGAGAGCGGTTC
  961 ---------+---------+---------+---------+---------+---------+ 1020
      AGGGTGGGGACCGTCCGACGGTAGAAACGGTTCGTGTCCTCCAGCGGGCCTCTCGCCAAG

   a: SerHisProTrpGlnAlaAlaIlePheAlaLysHisArgArgSerProGlyGluArgPhe -
```

Abb. 1c

```
          CTGTGCGGGGGCATACTCATCAGCTCCTGCTGGATTCTCTCTGCCGCCCACTGCTTCCAG
     1021 ---------+---------+---------+---------+---------+---------+ 1080
          GACACGCCCCCGTATGAGTAGTCGAGGACGACCTAAGAGAGACGGCGGGTGACGAAGGTC

       a: LeuCysGlyGlyIleLeuIleSerSerCysTrpIleLeuSerAlaAlaHisCysPheGln -

          GAGAGGTTTCCGCCCCACCACCTGACGGTGATCTTGGGCAGAACATACCGGGTGGTCCCT
     1081 ---------+---------+---------+---------+---------+---------+ 1140
          CTCTCCAAAGGCGGGGTGGTGGACTGCCACTAGAACCCGTCTTGTATGGCCCACCAGGGA

       a: GluArgPheProProHisHisLeuThrValIleLeuGlyArgThrTyrArgValValPro -

          GGCGAGGAGGAGCAGAAATTTGAAGTCGAAAAATACATTGTCCATAAGGAATTCGATGAT
     1141 ---------+---------+---------+---------+---------+---------+ 1200
          CCGCTCCTCCTCGTCTTTAAACTTCAGCTTTTTATGTAACAGGTATTCCTTAAGCTACTA

       a: GlyGluGluGluGlnLysPheGluValGluLysTyrIleValHisLysGluPheAspAsp -

          GACACTTACGACAATGACATTGCGCTGCTGCAGCTGAAATCGGATTCGTCCCGCTGTGCC
     1201 ---------+---------+---------+---------+---------+---------+ 1260
          CTGTGAATGCTGTTACTGTAACGCGACGACGTCGACTTTAGCCTAAGCAGGGCGACACGG

       a: AspThrTyrAspAsnAspIleAlaLeuLeuGlnLeuLysSerAspSerSerArgCysAla -

          CAGGAGAGCAGCGTGGTCCGCACTGTGTGCCTTCCCCCGGCGGACCTGCAGCTGCCGGAC
     1261 ---------+---------+---------+---------+---------+---------+ 1320
          GTCCTCTCGTCGCACCAGGCGTGACACACGGAAGGGGGCCGCCTGGACGTCGACGGCCTG

       a: GlnGluSerSerValValArgThrValCysLeuProProAlaAspLeuGlnLeuProAsp -

          TGGACGGAGTGTGAGCTCTCCGGCTACGGCAAGCATGAGGCCTTGTCTCCTTTCTATTCG
     1321 ---------+---------+---------+---------+---------+---------+ 1380
          ACCTGCCTCACACTCGAGAGGCCGATGCCGTTCGTACTCCGGAACAGAGGAAAGATAAGC

       a: TrpThrGluCysGluLeuSerGlyTyrGlyLysHisGluAlaLeuSerProPheTyrSer -

          GAGCGGCTGAAGGAGGCTCATGTCAGACTGTACCCATCCAGCCGCTGCACATCACAACAT
     1381 ---------+---------+---------+---------+---------+---------+ 1440
          CTCGCCGACTTCCTCCGAGTACAGTCTGACATGGGTAGGTCGGCGACGTGTAGTGTTGTA

       a: GluArgLeuLysGluAlaHisValArgLeuTyrProSerSerArgCysThrSerGlnHis -

          TTACTTAACAGAACAGTCACCGACAACATGCTGTGTGCTGGAGACACTCGGAGCGGCGGG
     1441 ---------+---------+---------+---------+---------+---------+ 1500
          AATGAATTGTCTTGTCAGTGGCTGTTGTACGACACACGACCTCTGTGAGCCTCGCCGCCC

       a: LeuLeuAsnArgThrValThrAspAsnMetLeuCysAlaGlyAspThrArgSerGlyGly -

          CCCCAGGCAAACTTGCACGACGCCTGCCAGGGCGATTCGGGAGGCCCCCTGGTGTGTCTG
     1501 ---------+---------+---------+---------+---------+---------+ 1560
          GGGGTCCGTTTGAACGTGCTGCGGACGGTCCCGCTAAGCCCTCCGGGGGACCACACAGAC

       a: ProGlnAlaAsnLeuHisAspAlaCysGlnGlyAspSerGlyGlyProLeuValCysLeu -
```

Abb. 1d

```
     AACGATGGCCGCATGACTTTGGTGGGCATCATCAGCTGGGGCCTGGGCTGTGGACAGAAG
1561 ---------+---------+---------+---------+---------+---------+ 1620
     TTGCTACCGGCGTACTGAAACCACCCGTAGTAGTCGACCCCGGACCCGACACCTGTCTTC
```

a:   AsnAspGlyArgMetThrLeuValGlyIleIleSerTrpGlyLeuGlyCysGlyGlnLys -

```
     GATGTCCCGGGTGTGTACACCAAGGTTACCAACTACCTAGACTGGATTCGTGACAACATG
1621 ---------+---------+---------+---------+---------+---------+ 1680
     CTACAGGGCCCACACATGTGGTTCCAATGGTTGATGGATCTGACCTAAGCACTGTTGTAC
```

a:   AspValProGlyValTyrThrLysValThrAsnTyrLeuAspTrpIleArgAspAsnMet -

```
     CGACCGTGA
1681 --------- 1689
     GCTGGCACT
```

a:   ArgProEnd -

# Abb.2

# Abb. 3

pUCtPA

- Bst E II
- Sal I
- Hind III
- tPA
- Bgl II
- Sal I
- BamHI
- pUC

1) Bst E II – Hind III

2) +Oligonukleotide  MN 29/30 oder MN 31/32

3) Ligation

pUCtPA – CTX bzw.– CTN

- Bst E II
- XhoI bzw. NcoI
- Hind III
- tPA
- BamHI
- pUC

EP 0 350 692 A1

# Abb.4

(4a)

pUCtPA - CTX  →[XhoI]→  

MN 33/36

tPA +
Insertion

XhoI
XhoI — BamHI
HIII

BamHI

pUCtPA - CT T

pUC

(4b)

pUCtPA - CTX  →[XhoI + Hind III]→

MN 42/43

tPA +
Insertion

XhoI
HIII

BamHI

pUCtPA - CTFg

pUC

pUCtPA - CTX  →[XhoI + Hind III]→

MN 47/48
oder  MN 60-63
oder  MN 64/65

tPA+
Insertion

XhoI
HIII — BamHI

BamHI

pUCtPA - CTaF
bzw. - CTM
bzw. - CTg2F

pUC

0050/40 044    455/88

4c

pUCtPA – CTN $\xrightarrow[\text{Hind III}]{\text{XhoI +}}$

MN 40/41
oder MN 78-81

tPA +
Insertion

NcoI
HIII

BamHI

pUCtPA – CTV
bzw. – CTH

pUC

# Abb.5

455/88

# Abb.6

# Abb. 7

Abbildung 8

$$
\begin{array}{c}
[\text{Gal}(\alpha 1-3)]_{1-2} \\
[\text{Sia}(\alpha 2-3/6)]_{1-2}
\end{array}
\left[
\begin{array}{l}
\text{Gal}(\beta 1-4)\ \text{GlcNAc}(\beta 1-2)\ \text{Man}(\alpha 1-6) \\
\quad \underline{6'} \qquad\qquad \underline{5'} \qquad\qquad\quad \underline{4'} \\[4pt]
\text{Gal}(\beta 1-4)\ \text{GlcNAc}(\beta 1-2)\ \text{Man}(\alpha 1-3) \\
\quad \underline{6} \qquad\qquad \underline{5} \qquad\qquad\quad \underline{4} \\[4pt]
\text{Gal}(\beta 1-4)\ \text{GlcNAc}(\beta 1-4) \\
\quad \underline{8} \qquad\qquad \underline{7}
\end{array}
\right.
$$

Fuc$(\alpha 1-6)$

Man$(\beta 1-4)$ GlcNAc$(\beta 1-4)$ GlcNAc$(\beta 1-$N$-$Asn

$\underline{3}$ $\qquad$ $\underline{2}$ $\qquad$ $\underline{1}$

| | | |
|---|---|---|
| Gal | = | Galaktose |
| Man | = | Mannose |
| GlcNAc | = | N-Acetylglucosamin |
| Fuc | = | Fucose |
| Sia | = | Sialinsäure |
| Asn | = | Asparagin |

EP 0 350 692 A1

O.Z. 0050/40044

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 227 462 (CHIRON CORP. et al.) <br> * Seite 5, Zeilen 10-24; Ansprüche 1,2,14,24,31 * <br> ----- | 1-4 | C 12 N 15/00 <br> C 12 N 9/64 |

RECHERCHIERTE SACHGEBIETE (Int. Cl.5)

C 12 N

Der vorliegende Recherchenbericht wurde für alle Patentansprüche erstellt

| Recherchenort | Abschlußdatum der Recherche | Prüfer |
|---|---|---|
| DEN HAAG | 13-09-1989 | HUBER-MACK A. |